# EUROPEAN PATENT APPLICATION

(11) **EP 4 245 228 A1**
(43) Date of publication of application: **20.09.2023**
(21) Application number: 22730353.4
(22) Date of filing: 24.01.2022
(51) Int. Cl.: A61B 17/12

(54) **HEMOSTATIC CLIPS AND TISSUE CLIPPING DEVICE**

(71) Applicant: Jiangsu Vedkang Medical Science & Technology Co., Ltd, Changzhou, Jiangsu 213149 (CN)
(72) Inventor: MIAO, Donglin, Changzhou, Jiangsu 213149 (CN)
(74) Representative: Hermann, Felix
(86) International application number: PCT/CN2022/073433
(87) International publication number: WO 2023/137745

(57) **Abstract**

A hemostatic clip (10) is provided. The hemostatic clip (10) includes a first sleeve (10) provided with a first position-limiting part (12) at its head end; a clip body (20) movably provided in the first sleeve (10) and provided with a second position-limiting part (22) and a third position-limiting part (21) spaced apart from each other, and the second position-limiting part (22) selectively cooperating with the first position-limiting part (12) in a position-limiting way; and a position-limiting member (30) provided on the first sleeve (10) and provided with a fourth position-limiting part (31). The fourth position-limiting part (31) cooperates with the third position-limiting part (21) in a position-limiting way when the second position-limiting part (22) cooperates with the first position-limiting part (12) in a position-limiting way. A tissue clipping device is further provided.

## Description

### TECHNICAL FIELD

The present disclosure relates to the field of medical devices, and more particularly, to a hemostatic clip and a tissue clipping device.

### BACKGROUND

Hemorrhage is a common complication in internal medicine, such as angiorrhexis and angiorhagia, the polyp ligation hemorrhage, and the ulcer complication hemorrhage and other iatrogenic hemorrhages. In the past, treatment methods of hemorrphage mainly include blood transfusion and injection of coagulation drug, and serious hemorrhage needs to be treated by an emergency surgery. With the further maturity of the endoscopic technology, using a hemostatic clip to stop bleeding has become a main way for hemostasis under the endoscope. The hemostatic clip adopts the mechanic force generated by the close of two clipping arms to apply a pressure on the bleeding sites so as to block the blood flow. Then, the clipping arms are controlled by and then are separated from a controlling handle outside a human body, and thus the clipping arms are left inside the human body to achieve a lasting hemostatic effect. Since the hemostasis is achieved in a mechanical manner, it has advantages including no damage to adjacent tissues, low incidence rate of re-bleeding and low incidence rate of adverse reactions, etc.

In the related art, the hemostatic clip has common disadvantages including small clipping force of the clipping arms and un-reliable positioning, causing the installation and disassembly of the hemostatic clip to be troublesome and thus causing the surgery procedure to be complicated.

### SUMMARY

The present disclosure aims to solve at least one of the technical problems in the prior art. To this end, the present disclosure provides a hemostatic clip. In the hemostatic clip, a first position-limiting part limits a second position-limiting part in a direction opposite to a direction in which a fourth position-limiting part limits a third position-limiting part, such that the clip body is fixed on the first sleeve in the axial direction, improving the clipping stability of the clip body.

The present disclosure further provides another hemostatic clip.

The present disclosure further provides a tissue clipping device.

An embodiment in a first aspect of the present disclosure provides a hemostatic clip. The hemostatic clip includes a first sleeve, a clip body, and a position-limiting member. The first sleeve is provided with a first position-limiting part at its head end. The clip body is movably provided in the first sleeve and is provided with a second position-limiting part and a third position-limiting part in its axial direction. The second position-limiting part and the third position-limiting part are spaced apart from each other, and the second position-limiting part selectively cooperates with the first position-limiting part in a position-limiting way. The position-limiting member is provided on the first sleeve and is provided with a fourth position-limiting part. The fourth position-limiting part cooperates with the third position-limiting part in a position-limiting way when the second position-limiting part cooperates with the first position-limiting part in a position-limiting way.

In the hemostatic clip of the embodiments of the present disclosure, the first position-limiting part cooperates with the second position-limiting part in a position-limiting way, the third position-limiting part cooperates with the fourth position-limiting part in a position-limiting way, and the first position-limiting part limits the second position-limiting part in a direction opposite to that in which the fourth position-limiting part limits the third position-limiting part, such that the clip body is fixed on the first sleeve in the axial direction, improving the clipping stability of the clip body.

According to some embodiments of the present disclosure, the first position-limiting part limits the second position-limiting part in a direction opposite to a direction in which the fourth position-limiting part limits the third position-limiting part

According to some embodiments of the present disclosure, the first position-limiting part is configured as a first position-limiting step, the second position-limiting part is configured as a second position-limiting step, and the first position-limiting step cooperates with the second position-limiting step in a position-limiting way.

According to some embodiments of the present disclosure, the first sleeve includes a sleeve body and a position-limiting boss. The position-limiting boss is provided at the head end of the sleeve body, the position-limiting boss is located at at least one side of the clip body, and the first position-limiting step is formed between the position-limiting boss and the head end of the sleeve body.

According to some embodiments of the present disclosure, the clip body includes two clipping arms provided opposite to each other. Each of the two clipping arms includes a first clipping segment and a second clipping segment. The first clipping segment is provided in the first sleeve, the third position-limiting part is provided on the first clipping segment, and the first clipping segment has a width smaller than that of the second clipping segment to form the second position-limiting step at a connection between the first clipping segment and the second clipping segment.

According to some embodiments of the present disclosure, the third position-limiting part is configured as a protrusion, the fourth position-limiting part is configured as a position-limiting rod, and the protrusion cooperates with the position-limiting rod in a position-limiting way.

According to some embodiments of the present disclosure, the clip body includes two clipping arms provided opposite to each other, and each of the two clipping arms is provided with the protrusion.

According to some embodiments of the present disclosure, the protrusion is configured as a barb, the barb is provided with a first position-limiting surface at one side closer to the second position-limiting part, and the barb is provided with an oblique guiding surface at another side away from the second position-limiting part.

According to some embodiments of the present disclosure, the third position-limiting part is provided at an end of the clip body and is located at an edge of at least one side of the end in a width direction of the end. The third position-limiting part is configured as a resilient protrusion. The fourth position-limiting part is configured as a position-limiting rod. The resilient protrusion cooperates with the position-limiting rod in a position-limiting way.

According to some embodiments of the present disclosure, two third position-limiting parts are provided, one of the two third position-limiting parts is provided on one side of the end of the clip body, and the other of the two third position-limiting parts is provided on another side of the end of the clip body.

According to some embodiments of the present disclosure, the first sleeve has an avoidance hole, and the fourth position-limiting part is provided at the avoidance hole.

According to some embodiments of the present disclosure, the position-limiting member is ring-shaped. At least two third position-limiting parts are provided, and the at least two third position-limiting parts are provided opposite to each other. Two avoidance holes are provided opposite to each other. The position-limiting member includes two position-limiting rods and two connecting rods, each of the two connecting rods is connected between the two position-limiting rods, and the two position-limiting rods are provided at the two avoidance holes respectively.

According to some embodiments of the present disclosure, the first sleeve is provided with two position-limiting grooves between the two avoidance holes, the two connecting rods are provided in the two position-limiting grooves respectively, and one of the two connecting rods is provided with a notch.

An embodiment in a second aspect of the present disclosure provides a hemostatic clip. The hemostatic clip includes a first sleeve, a clip body, and a position-limiting member. The first sleeve has an avoidance hole. The clip body is movably provided in the first sleeve and is provided with a position-limiting part. The position-limiting member is provided on the first sleeve and is provided partially in the avoidance hole. The position-limiting member is configured to cooperate with the position-limiting part in a position-limiting way when the clip body moves by a predetermined distance in an axial direction.

According to some embodiments of the present disclosure, the position-limiting part is configured as a protrusion, the position-limiting member is provided with a position-limiting rod, the position-limiting rod is provided in the avoidance hole, and the protrusion cooperates with the position-limiting rod in a position-limiting way.

According to some embodiments of the present disclosure, the protrusion is configured as a barb, the barb is provided with a position-limiting surface on one side closer to the head end of the clip body, and the barb is provided with an oblique guiding surface on another side of the clip body.

According to some embodiments of the present disclosure, the position-limiting part is provided at an end of the clip body and is located at an edge of at least one side of the end in a width direction of the end. The position-limiting part is configured as a resilient protrusion, the position-limiting member is provided with a position-limiting rod, the position-limiting rod is provided in the avoidance hole, and the resilient protrusion cooperates with the position-limiting rod in a position-limiting way.

According to some embodiments of the present disclosure, two position-limiting parts are provided, one of the two position-limiting parts is provided at one side of the end of the clip body, and the other of the two position-limiting parts is provided at another side of the end of the clip body.

According to some embodiments of the present disclosure, the position-limiting member is ring-shaped. At least two position-limiting parts are provided, and the at least two position-limiting parts are provided opposite to each other. Two avoidance holes are provided, and the two avoidance holes are provided opposite to each other. The position-limiting member includes two position-limiting rods and two connecting rods, each of the two connecting rods is connected between the two position-limiting rods, and the two position-limiting rods are provided at the two avoidance holes respectively.

An embodiment in a third aspect of the present disclosure provides a tissue clipping device. The tissue clipping device includes an applying device and the hemostatic clip according to any of above-mentioned embodiments. The clip body is detachably connected with the applying device.

Additional aspects and advantages of the present disclosure will be given partially in the following description, or become apparent partially from the following description, or can be learned from practice of the present disclosure.

### BRIEF DESCRIPTION OF DRAWINGS

The above and/or additional aspects and advantages of the present disclosure will become apparent and easy to understand from the description of embodiments below in conjunction with the accompanying drawings, in which:
FIG. 1 is a schematic diagram illustrating a structure of a hemostatic clip according to an embodiment of the present disclosure;
FIG. 2 is an exploded view of a hemostatic clip according to an embodiment of the present disclosure;
FIG. 3 is a sectional view of a hemostatic clip according to an embodiment of the present disclosure, which is obtained at an angle ;
FIG. 4 is a sectional view of a hemostatic clip according to an embodiment of the present disclosure, which is obtained at another angle;
FIG. 5 is a schematic diagram illustrating a structure of an clip body according to an embodiment of the present disclosure;
FIG. 6 is a schematic view illustrating a portion A of FIG. 5;
FIG. 7 is a schematic view illustrating a portion B of FIG. 5;
FIG. 8 is a schematic diagram illustrating a structure of a clip body according to another embodiment of the present disclosure;
FIG. 9 is a schematic diagram illustrating a structure of a first sleeve according to an embodiment of the present disclosure;
FIG. 10 is a schematic diagram illustrating a structure of a position-limiting member according to an embodiment of the present disclosure;
FIG. 11 is a schematic diagram illustrating a structure of a second sleeve according to an embodiment of the present disclosure;
FIG. 12 is a schematic diagram illustrating a structure of a controlling rod according to an embodiment of the present disclosure;
FIG. 13 is a sectional view illustrating a tissue clipping device in an initial state according to an embodiment of the present disclosure;
FIG. 14 is a sectional view illustrating the tissue clipping device in a first implanting stage according to an embodiment of the present disclosure;
FIG. 15 is a sectional view illustrating the tissue clipping device in a second implanting stage according to an embodiment of the present disclosure;
FIG. 16 is a sectional view illustrating the tissue clipping device in a third implanting stage according to an embodiment of the present disclosure; and
FIG. 17 is a sectional view illustrating the tissue clipping device in a fourth implanting stage according to an embodiment of the present disclosure.

### Reference numerals:

100: Hemostatic clip;
10: First sleeve; 11: Avoidance hole: 12: First position-limiting part; 13: Sleeve body; 14: Position-limiting boss; 15: Position-limiting groove; 20: Clip body; 21: Third position-limiting part; 211: First position-limiting surface: 212: Oblique guiding surface; 22: Second position-limiting part; 221: Second position-limiting surface; 23: Clipping arm; 231: First clipping segment; 232: Second clipping segment; 24: Matching member; 25: First clipping tooth; 26: Second clipping tooth; 30: Position-limiting member; 31: Fourth position-limiting part; 32: Position-limiting rod; 33: Connecting rod; 34: First notch; 40: Second sleeve; 41: Avoidance slot; 42: Accommodation boss; 43: Accommodation space; 44: Through hole; 50: controlling rod; 51: Second notch; 52: Snap ring; 53: Rod body; 54: Guiding surface; 200: Connection joint; 300: Spring tube; 400: Drag wire.

### DESCRIPTION OF EMBODIMENTS

Embodiments of the present disclosure will be described in further detail below. The embodiments described with reference to the drawings are exemplary. The embodiments of the present disclosure will be described in detail below.

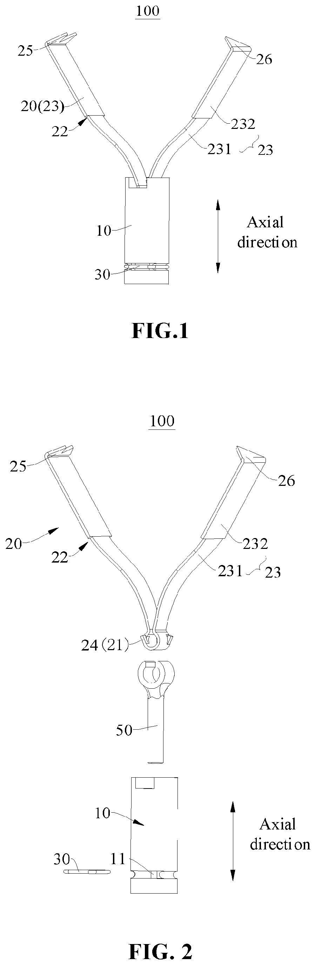

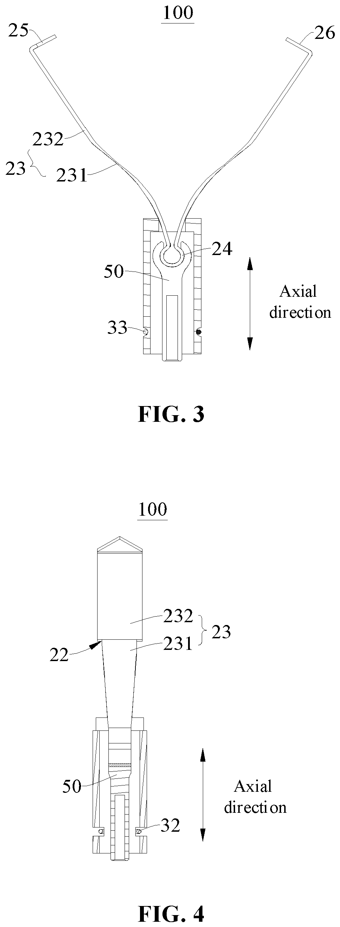

A hemostatic clip 100 according to embodiments in a first aspect of the present disclosure will be described below with reference to FIG. 1 to FIG. 17. The present disclosure further provides a tissue clipping device including the hemostatic clip 100.

As illustrated in FIG. 1 to FIG. 4, the hemostatic clip 100 according to embodiments in the first aspect of the present disclosure includes a first sleeve 10, a clip body 20 and a position-limiting member 30. The first sleeve 10 is provided with an avoidance hole 11, and is provided with a first position-limiting part 12 at its head end. The clip body 20 is movably provided in the first sleeve 10, the clip body 20 is provided in its axial direction with a second position-limiting part 22 and a third position-limiting part 21. The second position-limiting part 22 and the third position-limiting part 21 are spaced apart from each other. The second position-limiting part 22 selectively cooperates with the first position-limiting part 12 in a position-limiting way. The position-limiting member 30 is provided on the first sleeve 10, and is provided with a fourth position-limiting part 31. The fourth position-limiting part 31 is provided at the avoidance hole 11. When the second position-limiting part 22 cooperates with the first position-limiting part 12 in a position-limiting way, the fourth position-limiting part 31 cooperates with the third position-limiting part 21 in a position-limiting way. The third position-limiting part 21 may be a first axial position-limiting part, and the second position-limiting part 22 may be a second axial position-limiting part.

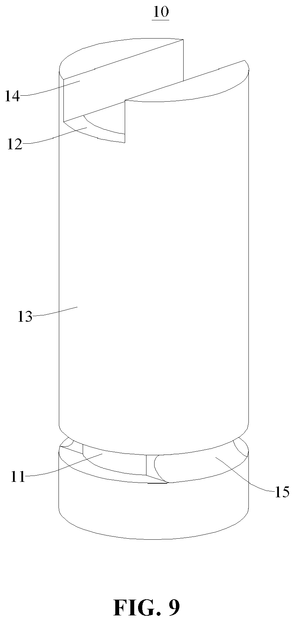

That is, with reference to FIG. 9, the first sleeve 10 is provided with the first position-limiting part 12, the clip body 20 is provided with the second position-limiting part 22, and the first position-limiting part 12 and the second position-limiting part 22 can cooperate with each other in a position-limiting way, so as to limit a degree of freedom of a clipping arm 23 in one direction. The clip body 20 is further provided with the third position-limiting part 21, the position-limiting member 30 is provided with the fourth position-limiting part 31, and the third position-limiting part 21 and the fourth position-limiting part 31 cooperate with each other in a position-limiting way, so as to limit a degree of freedom of the clipping arm 23 in another direction. In this way, the clip body 20 can be fixed on the first sleeve 10.

Further, the first position-limiting part 12 limits the second position-limiting part 22 in a direction opposite to a direction in which the fourth position-limiting part 31 limits the third position-limiting part 21. That is, the degree of freedom of an inward movement of the clip body 20 in the axial direction is limited by the position-limiting cooperation of the first position-limiting part 12 with the second position-limiting part 22, and the degree of freedom of an outward movement of the clip body 20 in the axial direction is limited by the position-limiting cooperation of the third position-limiting part 21 with the fourth position-limiting part 31. In this way, degrees of freedom of the clip body 20 in two directions along the axial direction are limited, that is, the clip body 20 will not move in the axial direction, so as to improve the clipping stability of the clip body 20.

Further, when the first position-limiting part 12 cooperates with the second position-limiting part 22 in a position-limiting way, the third position-limiting part 21 and the fourth position-limiting part 31 cooperates with each other in a position-limiting way. That is, an actual length between the first position-limiting part 12 and the fourth position-limiting part 31 is the same with an actual length between the third position-limiting part 21 and the second position-limiting part 22, and this length defines an effective clipping range for the hemostatic clip 100 in the embodiments of the present disclosure.

Therefore, through the arrangement in which the first position-limiting part 12 and the second position-limiting part 22 cooperate with each other in a position-limiting way, the third position-limiting part 21 and the fourth position-limiting part 31 cooperate with each other in a position-limiting way, and the first position-limiting part 12 limits the second position-limiting part 22 in a direction opposite to that in which the fourth position-limiting part 31 limits the third position-limiting part 21, the clip body 20 can be fixed on the first sleeve 10 in the axial direction, improving the clipping stability of the clip body 20.

In particular, referring to FIG. 1 to FIG. 6, the first position-limiting part 12 is configured as a first position-limiting step, the second position-limiting part 22 is configured as a second position-limiting step, and the first position-limiting step cooperates with the second position-limiting step in a position-limiting way. That is, through the position-limiting cooperation of the first position-limiting step with the second position-limiting step, the clip body 20 can cooperate with the first sleeve 10 in a position-limiting way. The position-limiting cooperation between the first position-limiting step and the second position-limiting step is more convenient and stable, thus improving the clipping stability of the clip body 20.

As illustrated in FIG. 9, the first sleeve 10 includes a sleeve body 13 and a position-limiting boss 14, the position-limiting boss 14 is provided at the head end of the sleeve body 13 and is located at at least one side of the clip body 20, and the first position-limiting step is formed between the position-limiting boss 14 and the head end of the sleeve body 13. Particularly, the first sleeve 10 includes the sleeve body 13 and the position-limiting boss 14, the sleeve body 13 is formed therein with a through hole, at least a portion of the clip body 20 is provided in the through hole, the position-limiting boss 14 is formed at one end of the sleeve body 13, and the first position-limiting step is formed between the position-limiting boss 14 and the head end of the sleeve body 13. In this way, when the clip body 20 cooperates with the first sleeve 10 in a position-limiting way, the second position-limiting step on the clip body 20 cooperates with the first position-limiting step formed by the position-limiting boss 14 in a position-limiting way, so as to limit the degree of freedom of the clip body 20 in one direction along the axial direction.

Two position-limiting bosses 14 may be provided at two sides of the through hole respectively. The two position-limiting bosses 14 cooperate with each other to form a penetration hole which is in communication with the through hole. The clip body 20 can extend through the penetration hole into the through hole. The two position-limiting bosses 14 may define the first position-limiting step together with the first sleeve 10. Further, the two position-limiting bosses 14 may limit an clipping angle of the clip body 20 from both sides of the clip body 20, so as to guarantee the clipping stability of the clip body 20.

As illustrated in FIG. 1 to FIG. 5 and FIG. 7 to FIG, 8, the clip body 20 includes two clipping arms 23 provided opposite to each other. Each of the two clipping arms 23 includes a first clipping segment 231 and a second clipping segment 232, the first clipping segment 231 is provided in the first sleeve 10, the second position-limiting part 22 is provided on the first clipping segment 231, and the first clipping segment 231 has a width smaller than that of the second clipping segment 232 to form the second position-limiting step at a connection between the first clipping segment 231 and the second clipping segment 232. In this way, the second position-limiting step is easy to form, which is convenient for manufacture of the clip body 20. Further, the strength of the second position-limiting step can be improved to make the position-limiting cooperation between the first position-limiting step and the second position-limiting step more stable. Of course, when each of the first clipping segment 231 and the second clipping segment 232 is a clipping segment with a non-constant width, it only requires that the first clipping segment 231 and the second clipping segment 232 have different sizes at the connection therebetween, that is, the end of the first clipping segment 231 closer to the second clipping segment 232 has a width smaller than that of the end of the second clipping segment 232 closer to the first clipping segment 231.

The two first clipping segments 231 can swing around a matching member 24. In this way, before the hemostatic clip 100 is implanted, the matching member 24 is in a natural state, and the two first clipping segments 231 are away from each other, and the two second clipping segments 232 are away from each other. Besides, each of the two second clipping segments 232 is provided with a clipping part at its side facing away from the first clipping segment 231. The two clipping parts are separate from each other. During a clipping process, the first clipping segments 231 shrink into the first sleeve 10, the angle between the two first clipping segments 231 becomes smaller and the angle between the two second clipping segments 232 becomes smaller, such that the clipping parts at the ends of the two second clipping segments 232 clip a human tissue to realize hemostasis. In addition, the first clipping segment 231 and the second clipping segment 232 both are rigid structures so as to stably clip the human tissue.

Further, as illustrated in FIG. 1 to FIG. 3 and FIG. 8, one clipping arm 23 is provided with a first clipping tooth 25 at its head end, and the other clipping arm 23 is provided with a second clipping tooth 26 at its head end, and the first clipping tooth 25 and the second clipping tooth 26 cooperate with each other. In this way, when the two clipping arms 23 get closer to each other, the first clipping tooth 25 and the second clipping tooth 26 cooperate with each other to clip the human tissue. Each of the first clipping tooth 25 and the second clipping tooth 26 may be triangular in shape, but is not limited to this. Each of the first clipping tooth 25 and the second clipping tooth 26 may be of another shape such as a rectangular or trapezoidal shape. There may be two first clipping teeth 25, and only one second clipping tooth 26. In this case, the second clipping tooth 26 is larger than each of the two first clipping teeth 25, and the second clipping tooth 26 can be engaged between the two first clipping teeth 25, such that the second clipping tooth 26 and the two first clipping teeth 25 can jointly clip the human tissue to guarantee the stability in clipping the human tissue.

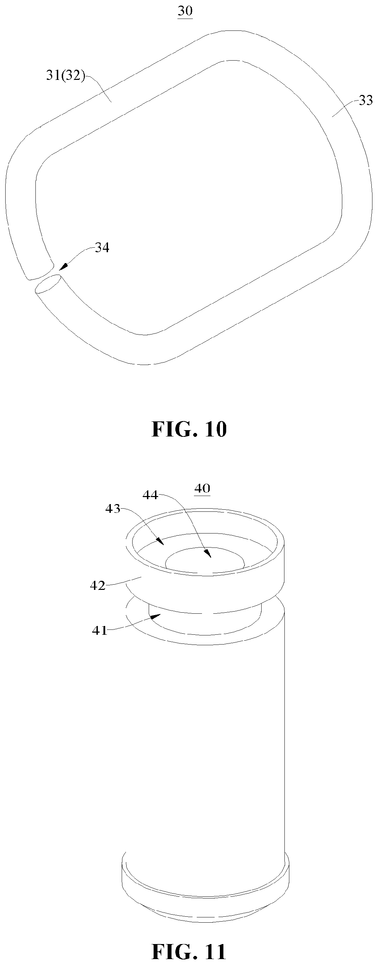

Further, referring to FIG. 9 and FIG. 10, the position-limiting member 30 is ring-shaped, at least two third position-limiting parts 21 may be provided, and at least two third position-limiting parts 21 are provided opposite to each other. Two avoidance holes 11 may be provided opposite to each other. The position-limiting member 30 includes two position-limiting rods 32 and two connection rods 33, each of the two connection rods 33 is connected between the two position-limiting rods 32, and the two position-limiting rods 32 are provided at the two avoidance holes 11 respectively. In other words, the two position-limiting rods 32 and the two connection rods 33 form a ring-shaped structure, such that the position-limiting member 30 can be sleeved on the first sleeve 10, and the two position-limiting rods 32 of the position-limiting member 30 can be fitted into the two avoidance holes 11 of the first sleeve 10, thereby facilitating the position-limiting cooperation of the protrusions with the position-limiting rods 32.

Further, the position-limiting member 30 can be formed by bending a metal rod or metal strip, which is convenient for manufacture of the position-limiting member 30. Alternatively, the position-limiting member 30 can be integrally manufactured through the injection molding or casting molding.

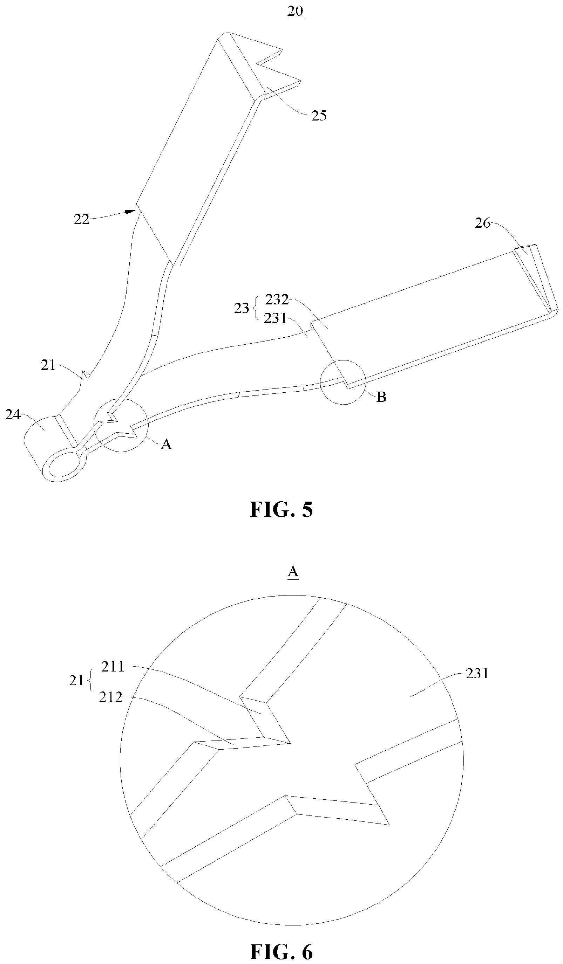

According to an optional embodiment of the present disclosure, as illustrated in FIG. 5 to FIG. 7, the clip body 20 includes two clipping arms 23 provided opposite to each other, each of the two clipping arms 23 is provided with the third position-limiting part 21, and the third position-limiting part 21 may be a protrusion. That is, each of the two clipping arms 23 provided opposite to each other is provided with the protrusion, such that the protrusion on each of the two clipping arms 23 could cooperate with the position-limiting rod 32 in a position-limiting way when the clip body 20 and the first sleeve 10 cooperate with each other in a position-limiting way, thereby further improving the stability of the position-limiting cooperation between the two clipping arms 23 and the first sleeve 10. In particular, both the two protrusions cooperate with a same position-limiting rod 32, facilitating the cooperation between the protrusions and the position-limiting rod 32 and reducing manufacture cost of the hemostatic clip 100.

Further, the clipping arms 23 of the clip body 20 can be relatively long, and thus the clip body 20 can clip a human tissue having a large bleeding site, so as to realize a better hemostatic effect.

Further, as illustrated in FIG. 6, the two clipping arms 23 each have protrusions on both sides thereof. In particular, each of the two clipping arms 23 is provided with a protrusion on each of its both sides. In this way, the clip body 20 is provided with four protrusions and all the four protrusions cooperate with the position-limiting rods 32, making the engagement of the clip body 20 onto the first sleeve 10 more stable.

In particular, as shown in FIG. 6, the protrusion may be barb-shaped. In other words, the barb-shaped protrusion can facilitate the movement of the protrusion in a direction facing away from the clipping part and avoid the movement of the protrusion in a direction towards the clipping part. That is, when the protrusion and the position-limiting rod 32 cooperate with each other in a position-limiting way, the clip body 20 can only move in a single direction. Similarly, the position-limiting cooperation between the first position-limiting step and the second position-limiting step can limit the movement of the second position-limiting step in a direction towards the protrusion and facilitate the movement of the second position-limiting step in a direction facing away from the protrusion. That is, after the first position-limiting step and the second position-limiting step cooperate with each other in a position-limiting way, the clip body 20 can only move in a single direction. Further, the direction in which the protrusion and the position-limiting rod 32 limit an degree of freedom is opposite to the direction in which the first position-limiting step and the second position-limiting step limit an degree of freedom, so as to avoid the movement of the clip body 20 in the axial direction.

In particular, as shown in FIG. 6, the barb is provided with a first position-limiting surface 211 at one side closer to the second position-limiting part 22, and the barb is provided with an oblique guiding surface 212 at another side away from the second position-limiting part 22. The first position-limiting surface 211 can realize the position-limiting cooperation of the protrusion with the position-limiting rod 32, and the oblique guiding surface 212 can facilitate the protrusion passing through the position-limiting rod 32. Further, the first position-limiting part 12 is provided with a second position-limiting surface 221 which is close to the third position-limiting part 21.

In this way, the degree of freedom of an inward movement of the clip body 20 in the axial direction is limited by the position-limiting cooperation of the first position-limiting step with the second position-limiting step, and the degree of freedom of an outward movement of the clip body 20 in the axial direction is limited by the position-limiting cooperation of the protrusion with the position-limiting rod 32. In this way, the clip body 20 will not move in the axial direction and can better clip a human tissue, so as to improve the clipping stability of the hemostatic clip 100. Further, the direction in which the protrusion and the position-limiting rod 32 limit an degree of freedom is opposite to the direction in which the first position-limiting step and the second position-limiting step limit an degree of freedom, such that limiting the degrees of freedom in the two directions can prevent the movement of the clip body 20 in the axial direction.

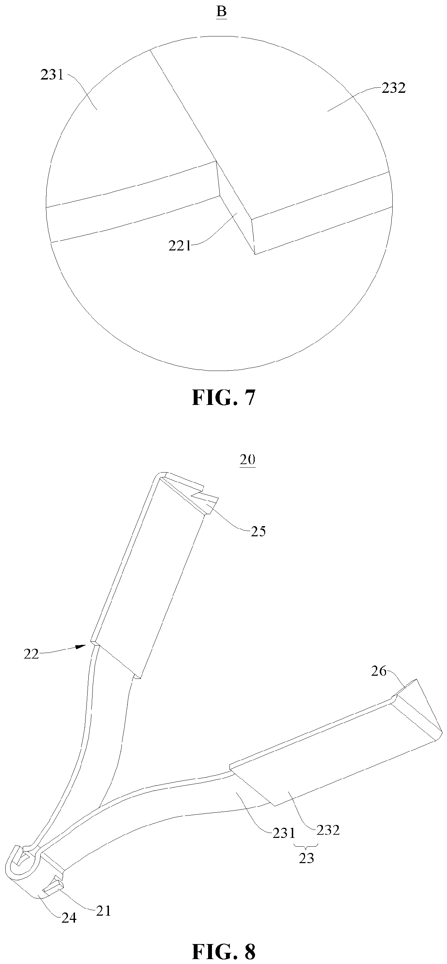

According to another optional embodiment of the present disclosure, as illustrated in FIG. 8, the clip body 20 further includes a matching member 24, an rear end of each of the two clipping arms 23 is connected onto the matching member 24. The matching member 24 is configured to cooperate with a controlling rod 50 of the tissue clipping device. The third position-limiting part 21 can be configured as a resilient protrusion provided on the matching member 24. When the controlling rod 50 and the matching member 24 cooperate with each other, the resilient protrusion is located at one side of the controlling rod 50 to avoid the controlling rod 50, such that the resilient protrusion will not affect the cooperation between the controlling rod 50 and the matching member 24.

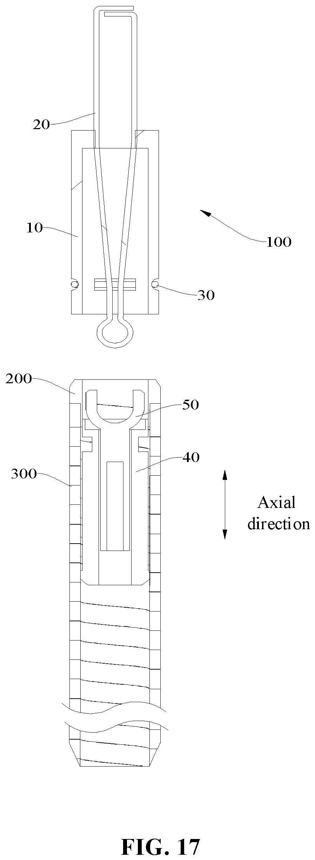

Further, in another embodiment of the present disclosure, the hardness of the matching member 24 may be different from the hardness of the clipping arm 23. Therefore, when the controlling rod 50 is separated from the matching member 24, the matching member 24 can deform e.g. from a major arc shape to a U shape, to realize the separation of the controlling rod 50 from the matching member 24. Of course, as illustrated in FIG. 17, when the controlling rod 50 is separated from the matching member 24, the controlling rod 50 may deform at its end, e.g. from a major arc shape to a U shape to realize the separation of the controlling rod 50 from the matching member 24. Both of the two above ways can achieve the separation of the controlling rod 50 from the matching member 24.

The third position-limiting part 21 may be provided on an edge of at least one side of the matching member 24 in a width direction of the matching member 24. That is, the third position-limiting part 21 is provided at the edge of the matching member 24 in the width direction of the matching member 24, facilitating the forming and popping out of the resilient protrusion.

In particular, the fourth position-limiting part 31 can be formed as a position-limiting rod 32. In this way, the position-limiting cooperation between the resilient protrusion and the position-limiting rod 32 can realize the position-limiting cooperation between the clip body 20 and the first sleeve 10. At least a portion of the position-limiting rod 32 can extend into the first sleeve 10 through the avoidance hole 11. When the resilient protrusion and the position-limiting rod 32 cooperate with each other in a position-limiting way, the resilient protrusion is located within the first sleeve 10, that is, the clip body 20 is further-back in the axial direction in comparison with the position where the clip body 20 is located in the un-clipping state.

In this way, the position-limiting cooperation of the resilient protrusion with the position-limiting rod 32 can limit the degree of freedom of the clip body 20 in one direction in the axial direction, and the position-limiting cooperation of the first position-limiting step with the second position-limiting step can limit the degree of freedom of the clip body 20 in another direction in the axial direction, such that the clip body 20 will not move in the axial direction and can be better clip a human tissue, thereby improving the clipping stability of the hemostatic clip 100.

The clipping arms 23 of the clip body 20 may be relatively short. In this way, when the space for clipping is limited, it is convenient for the claim body 20 to clip a human tissue.

In particular, the clip body 20 may be an integrally-formed structural member made of metal. For example, the clip body 20 may be formed by stamping a sheet metal or may be formed by casting or injection molding. In this way, on one hand, the manufacture of the clip body 20 can be relatively easy, facilitating the manufacture of the clip body 20. On the other hand, the integrally-formed clip body 20 has a higher structural strength, therefore the clip body 20 can clip a human tissue more stably.

The clip body 20 includes two clipping arms 23, and each of the two clipping arms 23 is provided with a protrusion on each of both sides in a width direction thereof. In this way, the protrusions on a same side of the two clipping arms 23 can be engaged with one position-limiting rod 32, and the protrusions on the other side of the two clipping arms 23 can be engaged with another position-limiting rod 32.

In particular, referring to FIG. 9 and FIG. 10, the first sleeve 10 is provided with two position-limiting grooves 15 between the two avoidance holes 11, the two connecting rods 33 are provided in the two position-limiting grooves 15 respectively, and one of the two connecting rods 33 is provided with a first notch 34. That is, the position-limiting grooves 15 are provided at the outer side of the first sleeve 10, and are provided between the two avoidance holes 11, so as to arrange the connecting rods 33 in the position-limiting grooves 15 and to fix the position-limiting member 30 onto the first sleeve 10. This can improve the stability of the position-limiting cooperation between the protrusions and the position-limiting rods 32, and improve the overall smoothness of the hemostatic clip 100. In this way, it can prevent exposure of the position-limiting member 30 and thus can prevent the position-limiting member 30 from interfering with operation of an operator, prevent mutual friction between the tissue clipping device and the channel of the endoscope during the tissue clipping device passes through the channel and any damage to the channel, and prevent an increased sliding resistance of the clip body 20 within the first sleeve 10 and any possible noise arising therefrom. Further, through the structure in which one of the two connecting rods 33 is provided with the first notch 34, it can not only facilitate the deform of the position-limiting member 30 so as to facilitate the controlling rod 50 and the second sleeve 40 passing through the position-limiting member 30, but also facilitate manufacture of the position-limiting member 30 e.g., manufacturing the position-limiting member 30 by bending a metal rod or metal strip.

As illustrated in FIG. 10, the connecting rod 33 is arc-shaped, and the position-limiting groove 15 is also arc-shaped. In this way, the connecting rod 33 can confirm to the shape of the outer side of the first sleeve 10, so as to further improve the overall smoothness of the hemostatic clip 100. The position-limiting rod 32 may be linear shaped to facilitate the position-limiting rod 32 extending into the first sleeve 10 and to facilitate the position-limiting cooperation of the protrusion with the position-limiting rod 32. Of course, the position-limiting rod 32 may be an arc-shaped rod having a certain curvature.

As illustrated in FIG. 2 to FIG. 4, FIG. 11 and FIG. 12, the hemostatic clip 100 further includes a second sleeve 40 and a controlling rod 50, a portion of the second sleeve 40 is provided within the first sleeve 10, a portion of the controlling rod 50 is provided within the second sleeve 40, and the controlling rod 50 and the clip body 20 are connected with each other. Another portion of the second sleeve 40 is provided within a spring tube 300 provided at the rear end of the first sleeve 10. When the clip body 20 is released, the second sleeve 40 is taken back together with the controlling rod 50.

Further, the controlling rod 50 can be fixed to a drag wire 400 on a handle by welding, such that the controlling rod 50 can be pushed forward or dragged backward in the second sleeve 40. The controlling rod 50 can be detachably connected to the clip body 20, such that when the controlling rod 50 is dragged, the controlling rod 50 can drive the clip body 20 to move in the axial direction.

Further, as illustrated in FIG. 11, the second sleeve 40 is provided with an avoidance groove 41 at one end thereof, and the position-limiting member 30 selectively cooperates with the avoidance groove 41 in a position-limiting way. In particular, when the controlling rod 50 and the front end of the second sleeve 40 are located within the first sleeve 10, and the controlling rod 50 and the clip body 20 cooperates with each other, the position-limiting member 30 and the avoidance groove 41 cooperates with each other in a position-limiting way so as to prevent the second sleeve 40 from moving within the first sleeve 10 in the axial direction and to prevent the second sleeve 40 from affecting the clipping of the clip body 20. Of course, the connection between the second sleeve 40 and the first sleeve 10 may not be totally fixed, and the second sleeve 40 may slightly move in the axial direction.

When the controlling rod 50 is dragged, the controlling rod 50 drives the clip body 20 to move backward in the axial direction. When the third position-limiting part 21 contacts with the position-limiting member 30, the third position-limiting part 21 pushes the position-limiting member 30 to spread out, that is, the two connecting rods 33 are separated from each other, and at this time the position-limiting member 30 stops its position-limiting action on the second sleeve 40. As the controlling rod 50 continues moving backward in the axial direction, the controlling rod 50 is separated from the clip body 20 and cooperates with the second sleeve 40. In this way, the controlling rod 50 and clip body 20 are separated from each other.

Further, as illustrated in FIG. 11, the second sleeve 40 is provided with an accommodation boss 42 at an end thereof, the accommodation boss 42 is formed therein with an accommodation space 43, a snap ring 52 is selectively provided in the accommodation space 43, the second sleeve 40 is formed therein with a through hole 44, the through hole 44 is in communication with the accommodation space 43, and a portion of the controlling rod 50 is provided in the through hole 44. That is, the end of the second sleeve 40 is provided with the accommodation boss 42, the accommodation boss 42 is provided with the accommodation space 43. In this way, during the process of the separation of the controlling rod 50 from the matching member 24, a portion of the controlling rod 50 can be engaged in the accommodation space 43, facilitating the separation of the controlling rod 50 from the matching member 24.

As illustrated in FIG. 5, FIG. 8 and FIG. 12, the controlling rod 50 is provided with a snap ring 52 at an end thereof, and the snap ring 52 has a second notch 51. The clip body 20 includes two clipping arms 23 provided opposite to each other, an arc-shaped matching member 24 is formed between the two clipping arms 23, and the matching member 24 is engaged in the snap ring 52. In this way, the arc-shaped matching member 24 is formed between the two clipping arms 23 of the clip body 20, and a distal end of the controlling rod 50 is provided with the snap ring 52 cooperating with the matching member 24. Further, the snap ring 52 is provided with the second notch 51, and the snap ring 52 is deformable, such that the matching member 24 can be engaged in the snap ring 52. In the actual application, the clip body 20 may involve two working phases, i.e., an repeated opening and closing phase in which a continuous connection is kept between the controlling rod 50 and the clip body 20, and a locking phase in which the controlling rod 50 drives the clip body 20 to move. In the locking phase, when it is determined that the clipping body 20 clips a human tissue tightly, the controlling rod 50 continues pulling the clip body 20 to cause the clip body 20 to move backward, so as to cause the snap ring 52 to deform and to be separated from the matching member 24. After that, when the clip body 20 is caused to continue to move backward by a certain distance, the snap ring 52 is separated from the matching member 24 so as to realize the separation of the clip body 20 from the controlling rod 50. The snap-fit between a protrusion and a groove can achieve a stable connection on one hand, and can cause separation of the protrusion from the groove as required.

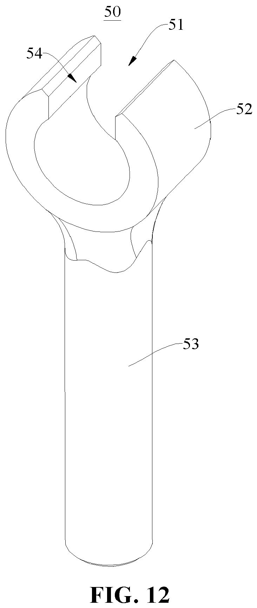

Besides, as illustrated in FIG. 12, the snap ring 52 is provided, at the second notch 51, with a guiding surface 54 for guiding the matching member 24. In this way, by providing the guiding surface 54 at the second notch 51 of the snap ring 52, it can provide a guiding function and facilitate the cooperation of the snap ring 52 with the matching member 24, improving the efficiency of the implant of the clip body 20 to some degree.

Further, as illustrated in FIG. 12, the guiding surface 54 can be an oblique guiding surface provided at each of the opposite sides of the second notch 54. In particular, the snap ring 52 can realize a guiding function by the oblique guiding surfaces, that is, facilitating the cooperation of the matching member 24 with the snap ring 52 through the oblique guiding surfaces. Further, the oblique guiding surfaces are provided at the two opposite sides of the second notch 51. In this way, both of the two opposite sides of the second notch 51 have the guiding function, so as to facilitate the connection between the matching member 24 and the snap ring 52.

Further, as illustrated in FIG. 12, the controlling rod 50 further includes a rod body 53, and the rod body 53 is provided at a side of the snap ring 52 facing away from the second notch 51 and has a diameter no larger than the width of the snap ring 52. In other words, the controlling rod 50 includes a rod body 53 and a snap ring 52, the snap ring 52 is configure to be connected with the clip body 20 and the rod body 53 is configured to be connected with the implanted component. The rod body 53 has a diameter no larger than the width of the snap ring 52, facilitating the implant of the clip body 20 and the pull out of the controlling rod 50. Further, the second sleeve 40 is provided therein with a through hole 44, and the rod body 53 is provided in the through hole 44.

A hemostatic clip 100 according to embodiments in a second aspect of the present disclosure will be described in details below.

As illustrated in FIG. 1 to FIG. 4, the hemostatic clip 100 according to the embodiments in the second aspect of the present disclosure includes a first sleeve 10, a clip body 20 and a position-limiting member 30. The first sleeve 10 has an avoidance hole 11. The clip body 20 is movably provided in the first sleeve, and is provided with a position-limiting part i.e. the above mentioned third position-limiting part 21. The position-limiting member 30 is provided on the first sleeve 10, and is partially provided in the avoidance hole 11. The position-limiting member 30 is configured to cooperate with the third position-limiting member 21 in a position-limiting way when the clip body 20 moves by a predetermined distance in the axial direction. In particular, the position-limiting member 30 is provided with a fourth position-limiting part 31 provided in the avoidance hole, and cooperates with the third position-limiting part 21 in a position-limiting way when the clip body 20 moves by a predetermined distance in the axial direction, i.e., when the clipping body 20 shrinks towards the interior of the first sleeve 10.

In this way, by the position-limiting cooperation between the third position-limiting part 21 of the clip body 20 and the fourth position-limiting part 31 of the position-limiting member 30, the hemostatic clip 100 can effectively limit the position of the clip body 20 when the clip body 20 shrinks towards the interior of the first sleeve 10, guaranteeing the clipping stability of the clip body 20 and the hemostasis effect of the hemostatic clip 100.

As illustrated in FIG. 5 to FIG. 7, the third position-limiting part 21 can be a protrusion, the position-limiting member 30 is provided with a position-limiting rod 32, the position-limiting rod 32 is provided at the avoidance hole 11, the protrusion cooperates with the position-limiting rod 32 in a position-limiting way. In particular, the position-limiting member 30 is ring-shaped, the clip body may be provided with at least two position-limiting parts 21, and the at least two position-limiting parts 21 may be provided opposite to each other, the first sleeve may be provided with two avoidance holes 11, and the two avoidance holes 11 are provided opposite to each other. The position-limiting member 30 includes two position-limiting rods 32 and two connecting rods 33, each of the two connecting rods 33 is connected between the two position-limiting rods 32, and the two position-limiting rods 32 are provided at the two avoidance holes 11 respectively. In other words, the two position-limiting rods 32 and the two connecting rods 33 form a ring shaped structure, such that the position-limiting member 30 can be sleeved on the first sleeve 10, and the two position-limiting rods 32 of the position-limiting member 30 can be fitted into the two avoidance holes 11 of the first sleeve 10, thereby facilitating the position-limiting cooperation of the protrusion with the position-limiting rod 32.

Referring to FIG. 6, the protrusion may be barb-shaped. In other words, the barb-shaped protrusion can facilitate the movement of the protrusion in a direction facing away from the clipping part and prevent movement of the protrusion in a direction towards the clipping part. In other words, the clip body 20 can only move in a single direction after the protrusion cooperates with the position-limiting rods 32 in a position-limiting way, guaranteeing the reliability of the position-limiting cooperation and further improving the stability of the hemostatic clip 100.

In particular, as illustrated in FIG. 6, the barb is provided with a position-limiting surface i.e. the above mentioned first position-limiting surface 211 at one side closer to the head end (the clipping part) of the clip body 20, the barb is provided with an oblique guiding surface 212 at another side. The first position-limiting surface 211 can realize the position-limiting cooperation of the protrusion with the position-limiting rod 32, and the oblique guiding surface 212 can facilitate the protrusion passing through the position-limiting rod 32. In this way, by position-limiting cooperation of the protrusion with the position-limiting rod 32, the clip body 20 can limit the degree of freedom of an outward movement of the clip body 20 in the axial direction, such that the clip body 20 will not move in the axial direction and can better clip a human tissue. That is, the clipping stability of the hemostatic clip 100 can be improved.

According to another optional embodiment of the present disclosure, as illustrated in FIG. 8, the clip body 20 further includes a matching member 24, a rear end of each of the two clipping arms 23 is connected onto the matching member 24. The matching member 24 is configured to cooperate with a controlling rod 50 of the tissue clipping device. The third position-limiting part 21 can be configured as a resilient protrusion provided on the matching member 24. When the controlling rod 50 cooperates with the matching member 24, the resilient protrusion is located at one side of the controlling rod 50 to avoid the controlling rod 50, such that the resilient protrusion will not affect the cooperation between the controlling rod 50 and the matching member 24.

The third position-limiting part 21 may be provided at an edge of at least one side of the matching member 24 in a width direction of the matching member 24. That is, the third position-limiting part 21 is provided at the edge of the matching member 24 in the width direction of the matching member 24, facilitating the forming and popping out of the resilient protrusion.

As illustrated in FIG. 8, two third position-limiting parts 21 are provided. One of the two third position-limiting parts 21 is provided at one side of an end of the clip body 20, and the other of the two third position-limiting parts 21 is provided at another side of the end of the clip body 20. That is, the two third position-limiting parts 21 are provided on both sides of the end of the hemostatic clip 20 respectively. In this way, the two third position-limiting parts 21 can effectively avoid the controlling rod 50 and will not interfere with the controlling rod 50, guaranteeing the position-limiting effect of the clip body 20.

A position-limiting assembly for tissue clipping according to embodiments in a third aspect of the present disclosure will be described in detail below.

The position-limiting assembly according to the embodiments in the third aspect of the present disclosure is configured to cooperate with the clip body 20 in a position-limiting way. The position-limiting assembly includes a first sleeve 10 and a position-limiting member 30. The first sleeve 10 has an avoidance hole 11. At least a portion of the clip body 20 is movably provided within the first sleeve 10. When the clip body 20 moves towards the interior of the first sleeve 10, the first sleeve 10 forces the clip body 20 to be in a closed and clipping state, that is, when the clip body 20 shrinks towards the interior of the first sleeve 10, the clip body 20 may be in a closed state for clipping a tissue.

The position-limiting member 30 is provided on the first sleeve 10, is partially provided at the avoidance hole 11, and is provided with a position-limiting part i.e. the fourth position-limiting part 31. The fourth position-limiting part 31 is provided at the avoidance hole 11, and is configured to cooperate with the clip body 20 in a position-limiting way. In particular, the clip body 20 may be provided with a third position-limiting part 21. When the clip body 20 moves towards the interior of the first sleeve 10, the third position-limiting part 21 and the fourth position-limiting part 31 can cooperate with each other in a position-limiting way. Therefore, limiting the position of the clip body 20 can be achieved and the clipping stability of the clip body 20 can be guaranteed.

In this way, such a position-limiting assembly enables the clip body 20 to cooperate with the position-limiting member 30 in a position-limiting way through the cooperation between the first sleeve 10 and the position-limiting member 30 when the clip body 20 is fixed on the position-limiting assembly, such that the clip body 20 can be fixed on the position-limiting assembly.

In particular, referring to FIG. 1, FIG. 2, FIG. 5 and FIG. 10, the position-limiting member 30 is ring-shaped, at least two third position-limiting parts 21 may be provided, and the at least two third position-limiting parts 21 are provided opposite to each other. Two avoidance holes 11 may be provided, and the two avoidance holes 11 are provided opposite to each other. The position-limiting member 30 includes two position-limiting rods 32 and two connecting rods 33, each of the two connecting rods 33 is connected between the two position-limiting rods 32, and the two position-limiting rods 32 are provided at the two avoidance holes 11 respectively. In other words, the two position-limiting rods 32 and the two connecting rods 33 form a ring shaped structure, such that the position-limiting member 30 can be sleeved on the first sleeve 10, and two position-limiting rods 32 of the position-limiting member 30 can be fitted into the two avoidance holes 11 of the first sleeve 10, thereby facilitating the position-limiting cooperation of the protrusions with the position-limiting rods 32.

Referring to FIG. 9 and FIG. 10, the first sleeve 10 is provided with two position-limiting grooves 15 between the two avoidance holes 11, the two connecting rods 33 are provided in the two position-limiting grooves 15 respectively. The ring-shaped position-limiting member 30 is provided with a first notch 34, in particular, one of the two connecting rods 33 is provided with the first notch 34. That is, the position-limiting grooves 15 are provided at the outer side of the first sleeve 10, and are provided between the two avoidance holes 11, so as to arrange the connecting rods 33 in the position-limiting grooves 15 to fix the position-limiting member 30 onto the first sleeve 10. This can improve the stability of the position-limiting cooperation between the protrusions and the position-limiting rods 32, and improve the overall smoothness of the hemostatic clip 100. In this way, it can prevent exposure of the position-limiting member 30 and thus can prevent the position-limiting member 30 from interfering with the operation of an operator, prevent mutual friction between the tissue clipping device and the channel of the endoscope during the tissue clipping device passes through the channel and any damage to the channel, and prevent an increased sliding resistance of the clip body 20 within the first sleeve 10 and any possible noise arising therefrom. Further, through the structure in which one of the two connecting rods 33 is provided with the first notch 34, it can not only facilitate the deform of the position-limiting member 30 so as to facilitate the controlling rod 50 and the second sleeve 40 passing through the position-limiting member 30, but also facilitate manufacture of the position-limiting member 30 e.g., manufacturing the position-limiting member 30 by bending a metal rod or metal strip.

As illustrated in FIG. 10, the connecting rod 33 is arc-shaped, and the position-limiting groove 15 is also arc-shaped. In this way, the connecting rod 33 can confirm to the shape of the outer side of the first sleeve 10, so as to further improve the overall smoothness of the hemostatic clip 100. The position-limiting rod 32 may be linear shaped to facilitate the position-limiting rod 32 extending into the first sleeve 10 and to facilitate the position-limiting cooperation of the protrusion with the position-limiting rod 32. Of course, the position-limiting rod 32 may be an arc-shaped rod having a certain curvature.

The hemostatic clip 100 according to the present disclosure includes the position-limiting assembly in the above mentioned embodiments and the clip body 20, and the fourth position-limiting part 31 selectively cooperates with the clip body 20 in a position-limiting way. The hemostatic clip having the position-limiting assembly configured as above has a better stability and a better hemostasis effect.

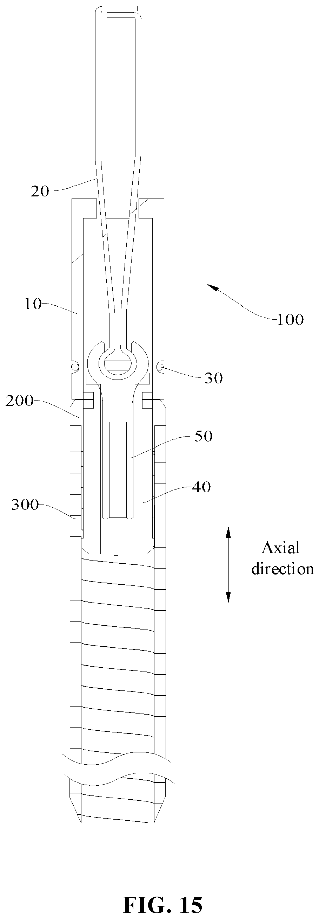

As illustrated in FIG. 15, the hemostatic clip 100 further includes a second sleeve 40 and a controlling rod 50, a portion of the second sleeve 40 is provided within the first sleeve 10, a portion of the controlling rod 50 is provided within the second sleeve 40, and the controlling rod 50 and the clip body 20 are connected with each other. Another portion of the second sleeve 40 is provided within a spring tube 300 provided at the rear end of the first sleeve 10. When the clip body 20 is released, the second sleeve 40 is taken back together with the controlling rod 50.

Further, as illustrated in FIG. 11, the second sleeve 40 is provided with an avoidance groove 41 at one end thereof, and the position-limiting member 30 selectively cooperates with the avoidance groove 41 in a position-limiting way. In particular, when the controlling rod 50 and the front end of the sleeve 40 are located within the first sleeve 10, and the controlling rod 50 and the clip body 20 cooperates with each other, the position-limiting member 30 and the avoidance groove 41 cooperates with each other in a position-limiting way so as to prevent the second sleeve 40 from moving within the first sleeve 10 in the axial direction and to prevent the second sleeve 40 from affecting the clipping of the clip body 20. Of course, the connection between the second sleeve 40 and the first sleeve 10 may not be totally fixed, and the second sleeve 40 may slightly move in the axial direction.

A tissue clipping device according to embodiments in a fourth aspect of the present disclosure includes an applying device and the hemostatic clip 100 according to any one of the above mentioned embodiments. The clip body 20 is detachably connected to the applying device. In this way, when the hemostatic clip 100 is in a tight clipping state, the applying device can be separated from the hemostatic clip 100 to leave the clip body to clip a human tissue. Further, through the arrangement in which the first position-limiting part 12 cooperates with the second position-limiting part 22 in a position-limiting way, the third position-limiting part 21 cooperates with the fourth position-limiting part 31 in a position-limiting way, and the first position-limiting part 12 limits the second position-limiting part 22 in a direction opposite to that in which the fourth position-limiting part 31 limits the third position-limiting part 21, the clip body 20 can be fixed on the first sleeve 10 in the axial direction, improving the clipping stability of the clip body 20.

As illustrated in FIG. 1 to FIG. 4, the applying device includes a connection joint 200, a spring tube 300, a drag wire 400 and a handle. The rear end of the first sleeve 10 is connected with the connection joint 200, and the head end of the spring tube 300 is connected with the connection joint 200. The controlling rod 50 extends through the second sleeve 40. When the controlling rod 50 cooperates with the matching member 24, the second sleeve 40 is provided among the first sleeve 10, the connection joint 200 and the spring tube 300, thus the positions of the first sleeve 10, the connection joint 200 and the spring tube 300 relative to each other can be maintained. The rear end of the controlling rod 50 is connected with the drag wire 400 and the proximal end of the drag wire 400 can be connected to the handle, such that the axial movement of the controlling rod 50 can be controlled by operation of the handle by the user.

The implanting process of the hemostatic clip 100 will be described with reference to FIG. 5 and FIG. 13 to FIG. 17.

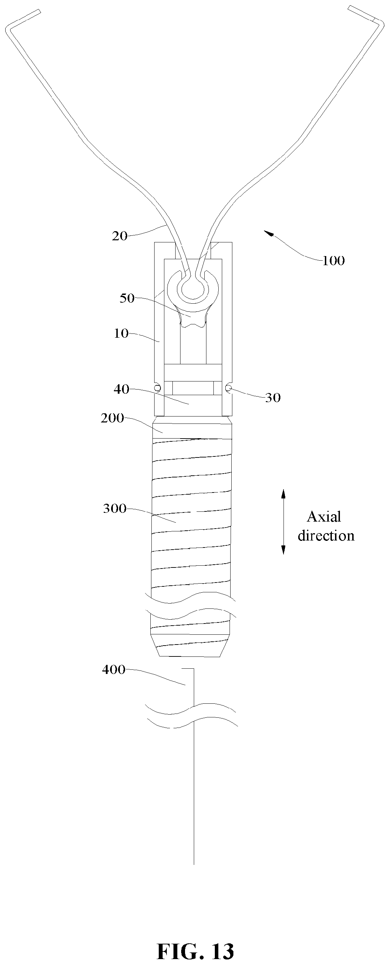

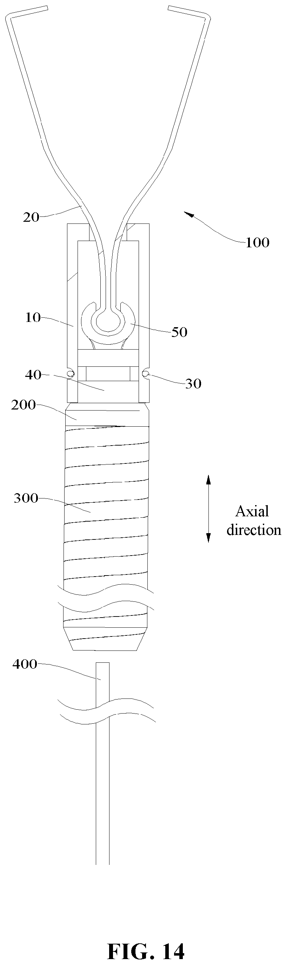

First, as illustrated in FIG. 13 and FIG. 14, the controlling rod 50 is pulled back by the drag wire 400, and the second sleeve 40 abuts against the controlling rod 50. In this stage, the third position-limiting part 21 and the first position-limiting part 12 have not reached their corresponding cooperative positions, i.e., have not been in cooperation with the position-limiting member 30 and the proximal end of the first sleeve 10. The controlling rod 50 has moved backward in the axial direction in comparison with the original position of the controlling rod 50.

Then, as illustrated in FIG. 15, the controlling rod 50 is kept being pulled by the drag wire 400, and the controlling rod 50 drives the second sleeve 40 and the clip body 20 to move backward in the axial direction, and the second sleeve 40 forces the position-limiting member 30 to expand so as to allow the proximal end of the second sleeve 40 and the controlling rod 50 to pass through the position-limiting member 30. In this stage, the controlling rod 50 is still kept being connected with the clip body 20, the angle between the two clipping arms 23 becomes small until the two clipping arms 23 clip a human tissue together, and the controlling rod 50 does not deform.

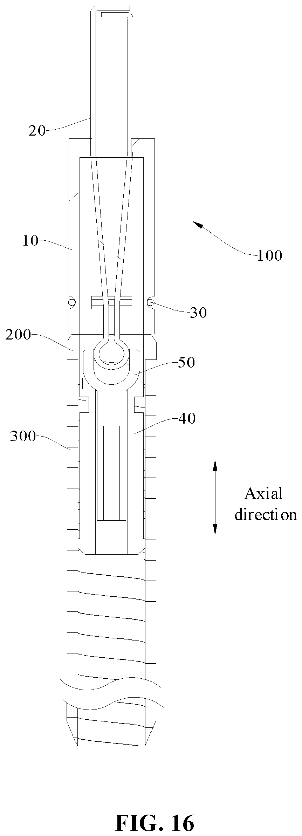

Then, as illustrated in FIG. 16, the controlling rod 50 is still kept being pulled by the drag wire 400. At this time, the protrusions are in a position-limiting cooperation with the position-limiting member 30. That is, the position-limiting member 30 slides through the oblique guiding surface 212 and then abuts against the first position-limiting surface 211. At the same time, the first position-limiting step and the second position-limiting step cooperate with each other in a position-limiting way, such that the clip body 20 is fixed on the first sleeve 10. In particular, the above two actions occur simultaneously. Further, the first clipping tooth 25 and the second clipping tooth 26 at the end of the clip body 20 cooperate with each other to realize clipping a human tissue.

Finally, as illustrated in FIG. 17, the operator continues pulling the drag wire 400 using the handle, the controlling rod 50 deforms from a major-arc shape to a U shape and is separated from the clip body 20. At this time, the clip body 20, the first sleeve 10 and the position-limiting member 30 are released, and the spring tube 300, the connection joint 200, the drag wire 400, the second sleeve 40 and the controlling rod 50 are pulled back.

Alternatively, the operator continues pulling the drag wire 400 by the handle, the matching member 24 of the clip body 20 deforms from a major-arc shape to a U shape, and the controlling rod 50 is separated from the clip body 20. At this time, the clip body 20, the first sleeve 10 and the position-limiting member 30 are released, and the spring tube 300, the connection joint 200, the drag wire 400, the second sleeve 40 and the controlling rod 50 are pulled back.

In the description of the present disclosure, it shall be understood that, terms such as "center", "longitudinal", "crosswise", "length", "width", "thickness", "up", "down", "front", "back", "left", "right", "vertical", "horizontal", "top", "bottom", "inside", "outside", "clockwise", "counter-clockwise", "axial", "radial", "circumferential" and others illustrating orientational or positional relations, are all on the basis of the orientational or positional relations illustrated in the drawings for convenience of simpleness of the description of the present disclosure, do not indicate or imply that the devices or elements must have a specific orientation or must be constructed and operated in a specific orientation and thus cannot be construed as limiting the present disclosure.

In the description of the present disclosure, the description with reference to the terms "one embodiment", "some embodiments", "exemplary embodiments", "example", "specific example", or "some examples", etc., means that specific features, structures, materials, or characteristics described in conjunction with the embodiment(s) or example(s) are included in at least one embodiment or example of the present disclosure. In the present disclosure, any illustrative reference to the above terms does not necessarily refer to the same embodiment(s) or example(s).

Although the embodiments of the present disclosure have been shown and described above, it can be appreciated by those of ordinary skill in the art that various changes, modifications, replacements and variants can be made to the above embodiments without departing from the principle and the spirit of the present disclosure. The scope of the disclosure is defined by claims and equivalents thereof.

## Claims

1. A hemostatic clip, comprising:
a first sleeve provided with a first position-limiting part at its head end;
a clip body movably provided in the first sleeve and provided in its axial direction with a second position-limiting part and a third position-limiting part, wherein the second position-limiting part and the third position-limiting part are spaced apart from each other, and the second position-limiting part selectively cooperates with the first position-limiting part in a position-limiting way; and
a position-limiting member provided on the first sleeve and provided with a fourth position-limiting part, wherein the fourth position-limiting part cooperates with the third position-limiting part in a position-limiting way when the second position-limiting part cooperates with the first position-limiting part in a position-limiting way.

2. The hemostatic clip according to claim 1, wherein the first position-limiting part limits the second position-limiting part in a direction opposite to a direction in which the fourth position-limiting part limits the third position-limiting part.

3. The hemostatic clip according to claim 1, wherein the first position-limiting part is configured as a first position-limiting step, the second position-limiting part is configured as a second position-limiting step, and the first position-limiting step cooperates with the second position-limiting step in a position-limiting way.

4. The hemostatic clip according to claim 3, wherein the first sleeve comprises a sleeve body and a position-limiting boss, the position-limiting boss is provided at the head end of the sleeve body, the position-limiting boss is located at at least one side of the clip body, and the first position-limiting step is formed between the position-limiting boss and the head end of the sleeve body.

5. The hemostatic clip according to claim 3, wherein the clip body comprises two clipping arms provided opposite to each other, each of the two clipping arms comprises a first clipping segment and a second clipping segment, the first clipping segment is provided in the first sleeve, the third position-limiting part is provided on the first clipping segment, and the first clipping segment has a width smaller than that of the second clipping segment to form the second position-limiting step at a connection between the first clipping segment and the second clipping segment.

6. The hemostatic clip according to claim 1, wherein the third position-limiting part is configured as a protrusion, the fourth position-limiting part is configured as a position-limiting rod, and the protrusion cooperates with the position-limiting rod in a position-limiting way.

7. The hemostatic clip according to claim 6, wherein the clip body comprises two clipping arms provided opposite to each other, and each of the two clipping arms is provided with the protrusion.

8. The hemostatic clip according to claim 6, wherein the protrusion is configured as a barb, the barb is provided with a first position-limiting surface at one side closer to the second position-limiting part, and the barb is provided with an oblique guiding surface at another side away from the second position-limiting part.

9. The hemostatic clip according to claim 1, wherein the third position-limiting part is provided at an end of the clip body and is located at an edge of at least one side of the end in a width direction of the end, the third position-limiting part is configured as a resilient protrusion, the fourth position-limiting part is configured as a position-limiting rod, and the resilient protrusion cooperates with the position-limiting rod in a position-limiting way.

10. The hemostatic clip according to claim 9, wherein two third position-limiting parts are provided, one of the two third position-limiting parts is provided on one side of the end of the clip body, and the other of the two third position-limiting parts is provided on another side of the end of the clip body.

11. The hemostatic clip according to claim 1, wherein the first sleeve has an avoidance hole, and the fourth position-limiting part is provided at the avoidance hole.

12. The hemostatic clip according to claim 11, wherein the position-limiting member is ring-shaped, at least two third position-limiting parts are provided, the at least two third position-limiting parts are provided opposite to each other, two avoidance holes are provided opposite to each other, the position-limiting member comprises two position-limiting rods and two connecting rods, each of the two connecting rods is connected between the two position-limiting rods, and the two position-limiting rods are provided at the two avoidance holes respectively.

13. The hemostatic clip according to claim 12, wherein the first sleeve is provided with two position-limiting grooves between the two avoidance holes, the two connecting rods are provided in the two position-limiting grooves respectively, and one of the two connecting rods is provided with a notch.

14. A hemostatic clip, comprising:
a first sleeve having an avoidance hole;
a clip body movably provided in the first sleeve and provided with a position-limiting part; and
a position-limiting member provided on the first sleeve and provided partially in the avoidance hole, wherein the position-limiting member is configured to cooperate with the position-limiting part in a position-limiting way when the clip body moves by a predetermined distance in an axial direction.

15. The hemostatic clip according to claim 14, wherein the position-limiting part is configured as a protrusion, the position-limiting member is provided with a position-limiting rod, the position-limiting rod is provided in the avoidance hole, and the protrusion cooperates with the position-limiting rod in a position-limiting way.

16. The hemostatic clip according to claim 15, wherein the protrusion is configured as a barb, the barb is provided with a position-limiting surface on one side closer to a head end of the clip body, and the barb is provided with an oblique guiding surface on another side of the clip body.

17. The hemostatic clip according to claim 14, wherein the position-limiting part is provided at an end of the clip body and is located at an edge of at least one side of the end in a width direction of the end, the position-limiting part is configured as a resilient protrusion, the position-limiting member is provided with a position-limiting rod, the position-limiting rod is provided in the avoidance hole, and the resilient protrusion cooperates with the position-limiting rod in a position-limiting way.

18. The hemostatic clip according to claim 17, wherein two position-limiting parts are provided, one of the two position-limiting parts is provided on one side of the end of the clip body, and the other of the two position-limiting parts is provided on another side of the end of the clip body.

19. The hemostatic clip according to claim 14, wherein the position-limiting member is ring-shaped, at least two position-limiting parts are provided, the at least two position-limiting parts are provided opposite to each other, two avoidance holes are provided, the two avoidance holes are provided opposite to each other, the position-limiting member comprises two position-limiting rods and two connecting rods, each of the two connecting rods is connected between the two position-limiting rods, and the two position-limiting rods are provided at the two avoidance holes respectively.

20. A tissue clipping device, comprising:
an applying device; and
the hemostatic clip according to any of claims 1 to 19, the clip body of the hemostatic clip being detachably connected with the applying device.
